# EUROPÄISCHE PATENTSCHRIFT

(11) **EP 3 628 345 B1**
(45) Veröffentlichungstag und Bekanntmachung des Hinweises auf die Patenterteilung: **05.10.2022**
(21) Anmeldenummer: 19190520.7
(22) Anmeldetag: 07.08.2019
(51) Int. Cl.: A61M 5/14, A61M 5/168

(54) **KIT ZUR MODULAREN AUSBILDUNG EINER MEDIZINISCHEN PUMPVORRICHTUNG SOWIE MEDIZINISCHE PUMPVORRICHTUNG**
KIT FOR MODULAR ASSEMBLY OF A MEDICAL PUMPING DEVICE AND MEDICAL PUMPING DEVICE
KIT POUR ASSEMBLAGE MODULAIRE D'UN DISPOSITIF DE POMPE A PERFUSION ET DISPOSITIF DE POMPE A PERFUSION

(30) Priorität: 26.09.2018 DE 102018216508
(43) Veröffentlichungstag der Anmeldung: 01.04.2020
(73) Patentinhaber: B. Braun Melsungen AG, 34212 Melsungen (DE)
(72) Erfinder: Haslbeck, Karsten, 34212 Melsungen (DE); Kent, Alexander David, 34327 Körle (DE); Stettner, Jens, 34212 Melsungen (DE)
(74) Vertreter: Patentanwälte Ruff, Wilhelm, Beier, Dauster & Partner mbB

(56) Entgegenhaltungen:
- US-A- 4 925 444
- US-A1- 2003 208 158
- US-A1- 2015 374 911
- US-A1- 2017 136 177

## Beschreibung

Die Erfindung betrifft ein Kit zur modularen Ausbildung einer medizinischen Pumpvorrichtung zur Förderung eines medizinischen Fluids sowie eine solche medizinische Pumpvorrichtung.

Medizinische Pumpvorrichtungen sind im Bereich der Medizintechnik allgemein bekannt und zur Verwendung bei einer Infusionstherapie vorgesehen. Die bekannte medizinische Pumpvorrichtung weist eine Pumpeinheit auf, die auch als elastomere Infusionspumpe oder medizinische Elastomerpumpe bezeichnet werden kann. Die Elastomerpumpe weist eine elastomere Membran auf, die ein Pumpvolumen zur Aufnahme und Förderung eines medizinischen Fluids ausbildet. Bei einer Befüllung des Pumpvolumens mit dem medizinischen Fluid wird die elastomere Membran ballonartig elastisch gedehnt. Die auf diese Weise gedehnte Membran bewirkt einen Förderdruck auf das Pumpvolumen. Mittels des Förderdrucks kann das medizinische Fluid beispielsweise in eine fluidleitend mit der Pumpvorrichtung verbindbare Katheterleitung gefördert und somit einem Patienten verabreicht werden. Die bekannte medizinische Pumpvorrichtung weist zudem eine fluidleitend mit dem Pumpvolumen verbundene Drosseleinheit auf. Die Drosseleinheit ist zur Drosselung der Förderung des medizinischen Fluids auf eine nominelle Flussrate eingerichtet und weist hierzu eine strömungsmechanische Drosselwirkung auf. Diese Drosselwirkung ist nicht veränderlich. Bei der bekannten medizinischen Pumpvorrichtung sind die Pumpeinheit und die Drosseleinheit fertigungsseitig unlösbar miteinander zusammengefügt. Hierdurch weist die bekannte medizinische Pumpvorrichtung eine festgelegte Spezifikation im Hinblick auf eine maximal verabreichbare Fluidmenge und eine nominelle Flussrate bei der Verabreichung des medizinischen Fluids auf. Dabei erfordern unterschiedliche medizinische Anwendungsfälle unterschiedliche maximal verabreichbare Fluidmengen und damit unterschiedlich dimensionierte Pumpvolumen sowie unterschiedliche nominelle Flussraten und damit unterschiedlich dimensionierte Drosselwirkungen. Dies führt bei der bekannten medizinischen Pumpvorrichtung dazu, dass diese in unterschiedlichsten Spezifikationen, d.h. mit großer Spezifikationsbreite, bereitgestellt werden muss. Hierdurch werden insbesondere die Fertigung und die Lagerhaltung erschwert.

Aus der US 2003/0208158 A1 ist ein Kit zur Ausbildung einer medizinischen Pumpvorrichtung bekannt, das eine manuelle Pumpe und eine motorisierte Pumpe sowie ein Druckinfusionsbeutelmodul zur Druckbeaufschlagung eines Infusionsbeutels aufweist. Je nach medizinischem Anwendungsfall kann entweder die manuelle Pumpe oder die motorisierte Pumpe mit dem Druckinfusionsbeutelmodul gekoppelt werden.

Aufgabe der Erfindung ist es, die Ausbildung einer medizinischen Pumpvorrichtung der eingangs genannten Art zu ermöglichen, wobei insbesondere eine vereinfachte Fertigung und eine vereinfachte Lagerhaltung unter Beibehaltung oder Erweiterung der Spezifikationsbreite ermöglicht sein sollen.

Diese Aufgabe wird durch ein Kit mit den Merkmalen des Anspruchs 1 gelöst.

Das erfindungsgemäße Kit ist zur modularen Ausbildung einer medizinischen Pumpvorrichtung zur Förderung eines medizinischen Fluids vorgesehen und weist auf
- eine Gruppe mit mehreren unterschiedlichen Pumpeinheiten, die jeweils ein Pumpvolumen zur Aufnahme und Förderung des medizinischen Fluids aufweisen,
- wobei die Pumpeinheiten unterschiedlich dimensionierte Pumpvolumina aufweisen,
- und wobei die Pumpeinheiten jeweils ein erstes Kupplungselement aufweisen, das derart gestaltet ist, dass das jeweilige Pumpvolumen fluidleitend mit einer Drosseleinheit verbindbar ist,
- und eine Gruppe mit mehreren unterschiedlichen Drosseleinheiten, die jeweils zur Drosselung der Förderung des medizinischen Fluids eingerichtet sind,
- wobei die Drosseleinheiten unterschiedlich dimensionierte Drosselwirkungen aufweisen,
- und wobei die Drosseleinheiten jeweils ein zweites Kupplungselement aufweisen, das derart gestaltet ist, dass die jeweilige Drosseleinheit fluidleitend mit einem Pumpvolumen einer der Pumpeinheiten verbindbar ist,
- wobei die Pumpeinheiten und die Drosseleinheiten räumlich voneinander getrennt angeordnet sind.

Durch die erfindungsgemäße Lösung kann - je nach medizinischen Anwendungsfall - eine medizinische Pumpvorrichtung mit einem für diesen Anwendungsfall erforderlichen Pumpvolumen und einer für diesen Anwendungsfall erforderlichen Flussrate ausgebildet werden. Diese Ausbildung der medizinischen Pumpvorrichtung unter Verwendung des erfindungsgemäßen Kits erfolgt in individualisierter Form endnutzerseitig und insoweit durch einen Patienten oder durch medizinisches Personal. Hierzu wählt die betreffende Person aus der Gruppe der unterschiedlichen Pumpeinheiten eine Pumpeinheit mit dem erforderlichen Pumpvolumen und aus der Gruppe der Drosseleinheiten eine Drosseleinheit mit der erforderlichen Drosselwirkung aus. Hiernach werden die ausgewählte Pumpeinheit und die ausgewählte Drosseleinheit mittels der Kupplungselemente, vorzugsweise unlösbar, fluidleitend miteinander verbunden. Hierdurch ermöglicht die erfindungsgemäße Lösung eine deutlich vereinfachte Fertigung und Lagerhaltung unter Beibehaltung der Spezifikationsbreite oder Erweiterung derselben. Denn es kann sowohl auf die Fertigung als auch auf die Lagerhaltung von medizinischen Pumpvorrichtungen mit unterschiedlichsten Spezifikationen im Hinblick auf eine festgelegte Kombination aus Pumpvolumen und Drosselwirkung verzichtet werden. Zudem ermöglicht die erfindungsgemäße Lösung eine dementsprechend vereinfachte Logistik, da eine reduzierte Anzahl von Artikeln und/oder ein reduziertes Transportvolumen transportiert werden muss. Ein erfindungsgemäßes Kit mit beispielsweise drei unterschiedlichen Pumpeinheiten und drei unterschiedlichen Drosseleinheiten weist sechs Teile auf und ermöglicht eine modulare Ausbildung einer medizinischen Pumpvorrichtung in neun unterschiedlichen Spezifikationen. Dabei sind lediglich sechs Teile - drei Pumpeinheiten und drei Drosseleinheiten - anstelle von neun unterschiedlich spezifizierten medizinischen Pumpvorrichtungen zu fertigen, zu transportieren und zu lagern. Ein erfindungsgemäßes Kit mit beispielsweise vier unterschiedlichen Pumpeinheiten und vier unterschiedlichen Drosseleinheiten weist acht Teile auf und ermöglicht eine modulare Ausbildung einer medizinischen Pumpvorrichtung in 16 unterschiedlichen Spezifikationen. Dabei sind lediglich acht Teile - vier Pumpeinheiten und vier Drosseleinheiten - anstelle von 16 unterschiedlich spezifizierten medizinischen Pumpvorrichtungen zu fertigen, zu transportieren und zu lagern. Ein erfindungsgemäßes Kit mit beispielsweise zehn unterschiedlichen Pumpeinheiten und zehn unterschiedlichen Drosseleinheiten weist 20 Teile auf und ermöglicht eine modulare Ausbildung einer medizinischen Pumpvorrichtung in 100 unterschiedlichen Spezifikationen. Dabei sind lediglich 20 Teile - 10 Pumpeinheiten und 10 Drosseleinheiten - anstelle von 100 unterschiedlich spezifizierten medizinischen Pumpvorrichtungen zu fertigen, zu transportieren und zu lagern. Die Pumpeinheiten sind vorzugsweise derart ausgebildet, dass eine Versorgung mit externer, insbesondere elektrischer, Betriebsenergie zur Förderung des medizinischen Fluids nicht notwendig ist. Vorzugsweise sind die Pumpeinheiten jeweils nach Art einer Elastomerpumpe ausgebildet. Elastomerpumpen sind im Bereich der Medizintechnik als solche grundsätzlich bekannt. Alternativ können die Pumpeinheiten über einen Gasdruck- oder Federspeicher mit Betriebsenergie versorgt werden. Weiter alternativ können die Pumpeinheiten als elektrische und/oder elektronische Pumpeinheiten gestaltet sein, wobei eine Versorgung mit elektrischer Betriebsenergie vorgesehen ist. Bei einer solchen Gestaltung kann die Pumpeinheit ein Fördervolumen aktiv steuern. Die Gruppe der unterschiedlichen Pumpeinheiten weist vorzugsweise eine Vielzahl unterschiedlicher Pumpeinheiten auf. Hierbei meint unterschiedlich baugleich und im Hinblick auf ein Fassungsvermögen des Pumpvolumens unterschiedlich dimensioniert. Deren Pumpvolumina sind vorzugsweise auf ein nominelles Fassungsvermögen zwischen 25 ml und 1000 ml dimensioniert. Beispielsweise können die Pumpvolumina unterschiedlich dimensioniert sein auf nominell 50 ml, 60 ml, 100 ml, 120 ml, 125 ml, 250 ml, 270 ml, 400 ml und/oder 500 ml. Die Drosseleinheiten wirken jeweils vorzugsweise strömungsmechanisch nach Art einer Flussdrossel. Flussdrosseln sind im Bereich der Strömungstechnik als solche grundsätzlich bekannt. Die Gruppe der unterschiedlichen Drosseleinheiten weist vorzugsweise eine Vielzahl von unterschiedlichen Drosseleinheiten auf. Hiermit meint unterschiedlich baugleich und im Hinblick auf die Drosselwirkung unterschiedlich dimensioniert. Die Drosselwirkung kann beispielsweise derart unterschiedlich dimensioniert sein, dass bei der Förderung des medizinischen Fluids eine nominelle Flussrate zwischen 0,5 ml/h und 1.000 ml/h bewirkt ist. Beispielsweise können die Drosselwirkungen unterschiedlich dimensioniert sein auf Flussraten von 2 ml/h, 4 ml/h, 5 ml/h, 10 ml/h, 50 ml/h, 100 ml/h, 175 ml/h, 200 ml/h, 250 ml/h und/oder 500 ml/h. Alternativ oder zusätzlich können die Drosselwirkungen der unterschiedlichen Drosseleinheiten unterschiedlich im Hinblick auf eine Toleranz und/oder Genauigkeit der jeweils erzielbaren Flussrate sein. Mit anderen Worten ausgedrückt, können die Drosseleinheiten unterschiedlich abgestufte Toleranzen und/oder Genauigkeiten der Drosselwirkung aufweisen. Die Drosselwirkung der Drosseleinheiten ist vorzugsweise unveränderlich im Sinne von nutzerseitig nicht einstellbar und/oder nicht steuerbar. Alternativ kann die Drosselwirkung mittels einer entsprechenden Stelleinrichtung einstellbar sein. Weiter alternativ können die Drosseleinheiten als elektrisch und/oder elektronisch steuerbare und/oder regelbare Drosseleinheiten gestaltet sein. Beispielsweise kann eine solche Drosseleinheit als Piezo-Element, das durch eine Kapazität in eine dedizierte Regelstellung gebracht wird, oder als elektronisch ansteuerbares Ventil gestaltet sein. Das erste Kupplungselement und das zweite Kupplungselement sind komplementär zueinander ausgebildet. In einem miteinander verbundenen Zustand bilden die Kupplungselemente eine fluidleitende Verbindung zwischen dem Pumpvolumen und der Drosseleinheit aus. Die Kupplungselemente können insbesondere als Steck- und/oder Schraubkonnektoren ausgebildet sein. Das erfindungsgemäße Kit sieht eine räumlich getrennte Anordnung der Pumpeinheiten und der Drosseleinheiten insoweit vor, als diese nicht fluidleitend miteinander verbunden sind. Die fluidleitende Verbindung erfolgt vielmehr erst endnutzerseitig, beispielsweise durch einen Patienten oder medizinisches Personal, bei einer modularen Ausbildung der medizinischen Pumpvorrichtung unter Verwendung des Kits. Vorzugsweise können die Pumpeneinheiten und die Drosseleinheiten zueinander komplementäre elektrische und/oder elektro-mechanische Schnittstellen aufweisen, über welche im verbundenen Zustand der Schnittstellen Informationen ausgetauscht werden können. Solche Informationen können beispielsweise eine Identifikationsnummer, eine Spezifikation oder dergleichen betreffen. Bei einem Kontaktieren der Schnittstellen kann zudem eine Art Entwertung erfolgen, sofern die Pumpeinheit und/oder die Drosseleinheit als Einmalartikel zur einmaligen Verwendung vorgesehen sind.

In Ausgestaltung der Erfindung weisen die Pumpeinheiten jeweils eine elastomere Membran auf, die das Pumpvolumen zur Aufnahme und Förderung des medizinischen Fluids ausbildet und in einem wenigstens teilweise mit dem medizinischen Fluid befüllten Zustand derart elastisch gedehnt ist, dass ein Förderdruck auf das Pumpvolumen bewirkt ist. Demnach sind die Pumpeinheiten jeweils nach Art einer Elastomerpumpe ausgebildet. Elastomerpumpen sind im Bereich der Medizintechnik als solche grundsätzlich bekannt und können auch als elastomere Infusionspumpen bezeichnet werden. Dabei ist die Pumpeinheit vorzugsweise derart dimensioniert, dass sie ohne weiteres von einem Patienten am Körper getragen und ohne eine externe Energieversorgung insbesondere im Rahmen einer ambulanten Infusionstherapie verwendbar ist. Die Energie zur Förderung des medizinischen Fluids wird vorzugsweise allein durch die elastisch gedehnte elastomere Membran bereitgestellt.

In weiterer Ausgestaltung der Erfindung sind das erste Kupplungselement und das zweite Kupplungselement derart komplementär gestaltet, dass eine mittels der Kupplungselemente bewirkte fluidleitende Verbindung unlösbar ist. Die Kupplungselemente dienen demnach einer einmaligen endnutzerseitigen Verbindung der jeweiligen Pumpeinheit mit der jeweiligen Drosseleinheit. Nach einer einmal hergestellten fluidleitenden Verbindung ist diese nicht mehr zerstörungsfrei lösbar. Diese Ausgestaltung der Erfindung wirkt insbesondere einem ungewollten Lösen der fluidleitenden Verbindung entgegen. Hierdurch kann eine verbesserte Patientensicherheit erreicht werden. Die Kupplungselemente können hierzu mit zueinander komplementären Rast- oder Schnappgeometrien versehen sein, die in verbundenem Zustand ein Lösen verhindern.

In weiterer Ausgestaltung der Erfindung weisen die Drosseleinheiten jeweils ein drittes Kupplungselement auf, das zur fluidleitenden Verbindung der Drosseleinheit und damit der medizinischen Pumpvorrichtung mit einem patientenseitigen Zugang eingerichtet ist. Vorzugsweise ist das dritte Kupplungselement stirnendseitig einer der Drosseleinheit zugeordneten Schlauchleitung angeordnet. Das dritte Kupplungselement ist vorzugsweise in Form eines im Bereich der Medizintechnik grundsätzlich bekannten Fluidkonnektors ausgebildet. Beispielsweise kann das dritte Kupplungselement in Form eines Luer-Konnektors oder eines NRFit-Konnektors ausgebildet sein.

In weiterer Ausgestaltung der Erfindung weisen die Drosseleinheiten jeweils ein Filterelement auf, das zur Filterung des medizinischen Fluids vorgesehen ist. Das Filterelement ist in einem fluidführenden Querschnitt der Drosseleinheit angeordnet. Mittels des Filterelements kann eine nochmals verbesserte Patientensicherheit erreicht werden, da etwaige partikelförmige Verunreinigungen des medizinischen Fluids bei dessen Förderung durch die Drosseleinheit herausgefiltert werden können. Alternativ oder zusätzlich können die Drosseleinheiten jeweils ein Indikatorelement ausweisen, das zur Anzeige eines Strömungszustands des medizinischen Fluids eingerichtet ist. Bspw. kann das Indikatorelement derart gestaltet sein, dass ein Fluss und ein Nicht-Fluss des Fluids und damit zwei unterschiedliche Strömungszustände anzeigbar sind.

In weiterer Ausgestaltung der Erfindung weisen die Pumpeinheiten jeweils einen Füllzustand auf, in dem das jeweilige Pumpvolumen mit dem medizinischen Fluid vorbefüllt ist. Vorbefüllt meint, dass das medizinische Fluid bereits bei der produktionsseitigen Bereitstellung des Kits in die Pumpvolumina eingefüllt wurde. Diese Ausgestaltung der Erfindung ist besonders vorteilhaft, wenn die Pumpeinheiten jeweils nach Art einer Elastomerpumpe ausgebildet sind und eine elastomere Membran aufweisen, die das Pumpvolumen ausbildet. Der vorbefüllte Füllzustand der Pumpeinheiten ermöglicht eine vereinfachte Handhabung des Kits, da auf eine nachträgliche, nutzerseitige Befüllung des Pumpvolumens verzichtet werden kann.

In weiterer Ausgestaltung der Erfindung weisen die Pumpeinheiten jeweils ein Versiegelungselement auf, mittels dessen das jeweilige Pumpvolumen öffenbar fluiddicht versiegelt ist. Das Versiegelungselement dient einem fluiddichten Verschließen des mit dem medizinischen Fluid vorbefüllten Pumpvolumens. Durch diese Versiegelung kann eine verbesserte Hygiene und letztlich eine verbesserte Patientensicherheit bei der Ausbildung der medizinischen Pumpvorrichtung unter Verwendung des Kits erreicht werden. Das Versiegelungselement kann insbesondere in Form einer Kappe, eines Stopfens, eines Schrauboder Drehverschlusses, eines Sollbruchelements oder dergleichen ausgebildet sein. Dabei kann die Versiegelung form-, kraft- und/oder stoffschlüssig bewirkt sein.

In weiterer Ausgestaltung der Erfindung weisen die Pumpeinheiten jeweils einen Druckminderer auf, mittels dessen ein Förderdruck des medizinischen Fluids reduzierbar ist. Der Druckminderer ist fluidleitend mit dem Pumpvolumen verbunden. Druckminderer sind im Bereich der Strömungstechnik als solche grundsätzlich bekannt. Der Druckminderer kann auch als Druckminderungsventil oder Reduzierventil bezeichnet werden. Mittels des Druckminderers wird gewährleistet, dass ein auslassseitig an der Pumpeinheit anliegender Druck des medizinischen Fluids einen bestimmten Grenzwert nicht überschreitet. Der Druckminderer wirkt insbesondere einer druckbedingten Überdosierung des medizinischen Fluids entgegen und ermöglicht letztlich eine nochmals verbesserte Patientensicherheit.

In weiterer Ausgestaltung der Erfindung ist ein weitere Gruppe mit mehreren unterschiedlichen Funktionseinheiten vorgesehen, wobei die Funktionseinheiten unterschiedlich dimensionierte, vorzugsweise strömungsmechanische, Eigenschaften aufweisen, und wobei die Funktionseinheiten jeweils fluidleitend mit einer der Pumpeinheiten und/oder einer der Drosseleinheiten verbindbar sind. Zur fluidleitenden Verbindung mit den Pumpeinheiten und/oder den Drosseleinheiten können die Funktionseinheiten jeweils mit entsprechenden Kupplungselementen versehen sein.

Die Erfindung betrifft zudem eine medizinische Pumpvorrichtung zur Förderung eines medizinischen Fluids, wobei die medizinische Pumpvorrichtung durch eine modulare Ausbildung unter Verwendung eines Kits nach der vorhergehenden Beschreibung gekennzeichnet ist.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematisch stark vereinfachter Darstellung eine Ausführungsform einer erfindungsgemäßen medizinischen Pumpvorrichtung, die unter Verwendung einer Ausführungsform eines erfindungsgemäßen Kits (Fig. 4) modular ausgebildet ist,
- Fig. 2: in schematisch stark vereinfachter Darstellung eine Pumpeinheit und eine Drosseleinheit der medizinischen Pumpvorrichtung nach Fig. 1 in einem unverbundenen Zustand,
- Fig. 3: in einer weiteren schematisch vereinfachten Darstellung die medizinische Pumpvorrichtung nach den Fig. 1 und 2, wobei weitere Gestaltungsmerkmale erkennbar und die Pump- und die Drosseleinheit voneinander getrennt dargestellt sind,
- Fig. 4: in schematisch stark vereinfachter Prinzipdarstellung eine Ausführungsform eines erfindungsgemäßen Kits, unter dessen Verwendung die medizinische Pumpvorrichtung nach den Fig. 1 bis 3 ausgebildet ist,
- Fig. 5: in einer Darstellungsweise entsprechend Fig. 4 eine Ausführungsform eines Kits mit wenigstens einer Pumpeinheit und wenigstens einer Drosseleinheit und
- Fig. 6: in schematisch stark vereinfachter Prinzipdarstellung eine Ausführungsvariante des Kits nach Fig. 4, wobei das Kit eine Gruppe mit unterschiedlichen Funktionseinheiten aufweist.

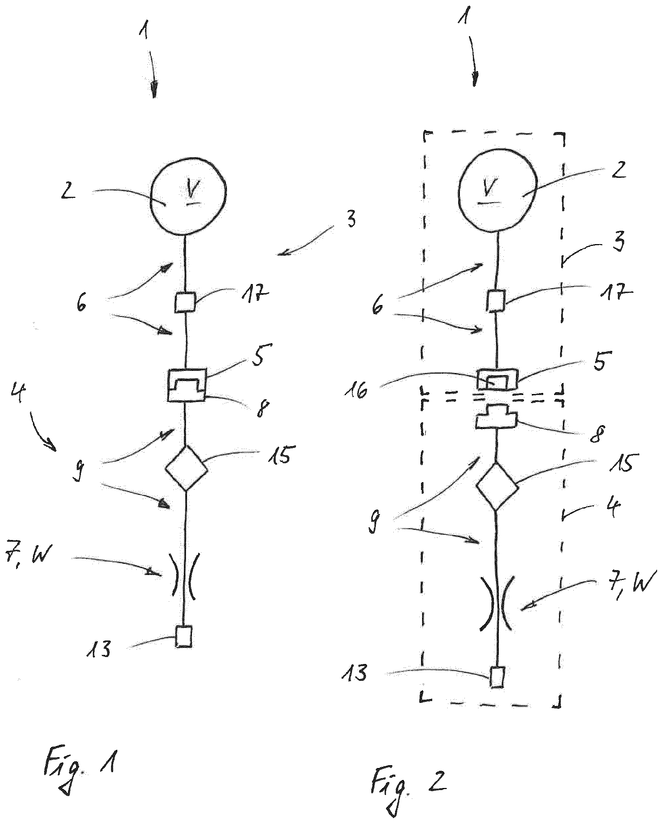

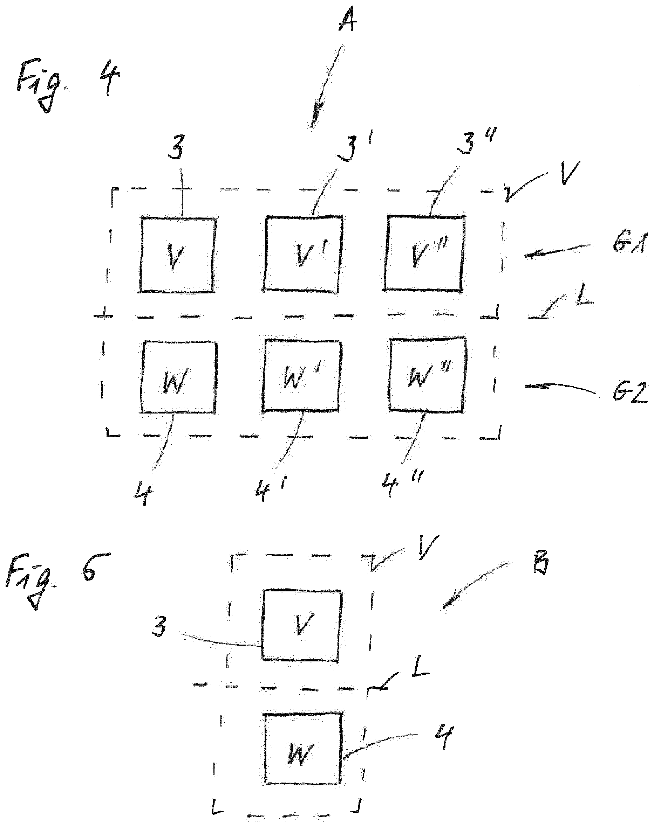

Eine medizinische Pumpvorrichtung 1 nach den Fig. 1 bis 3 ist zur Förderung eines medizinischen Fluids 2 im Rahmen einer ambulanten und/oder stationären Infusionstherapie vorgesehen. Die medizinische Pumpvorrichtung 1 ist auf noch näher beschriebene Weise unter Verwendung eines Kits A nach Fig. 4 modular ausgebildet. Nachfolgend wird zunächst auf die gegenständliche und funktionelle Ausgestaltung der medizinischen Pumpvorrichtung 1 näher eingegangen.

Die medizinische Pumpvorrichtung 1 weist eine Pumpeinheit 3 und eine Drosseleinheit 4 auf.

Die Pumpeinheit 3 weist ein Pumpvolumen V auf, das zur Aufnahme und Förderung des medizinischen Fluids 2 vorgesehen ist. Dabei ist die Pumpeinheit 3 auf noch näher beschriebene Weise derart gestaltet, dass das medizinische Fluid 2 ohne eine externe, insbesondere elektrische, Energieversorgung mittels der Pumpeinheit 3 förderbar ist. Die Pumpeinheit 3 weist zudem ein erstes Kupplungselement 5 auf. Das erste Kupplungselement ist derart gestaltet, dass das Pumpvolumen V fluidleitend mit der Drosseleinheit 4 verbindbar ist. Das erste Kupplungselement 5 ist vorliegend stirnendseitig einer Fluidleitung 6 angeordnet, die an einem dem ersten Kupplungselement 5 abgewandten Stirnende fluidleitend mit dem Pumpvolumen V verbunden ist.

Die Drosseleinheit 4 ist zur Drosselung der mittels der Pumpeinheit 3 bewirkten Förderung des medizinischen Fluids 2 eingerichtet. Hierzu weist die Drosseleinheit 4 ein Drosselelement 7 auf. Das Drosselelement 7 ist derart gestaltet, dass die Förderung des medizinischen Fluids 2 auf eine festgelegte Flussrate begrenzt ist. Hierzu weist das Drosselelement 7 eine entsprechende strömungstechnische Drosselwirkung W auf. Zudem weist die Drosseleinheit 4 ein zweites Kupplungselement 8 auf, das zur fluidleitenden Verbindung mit dem ersten Kupplungselement 5 der Pumpeinheit 3 vorgesehen ist. Zu diesem Zweck sind die Kupplungselemente 5, 8 komplementär zueinander ausgebildet. In dem anhand Fig. 1 ersichtlichen Zustand sind die Kupplungselemente 5, 8 fluidleitend miteinander verbunden. In dem anhand Fig. 2 ersichtlichen Zustand sind die Kupplungselemente 5, 8 demgegenüber voneinander getrennt. Das zweite Kupplungselement 8 ist stirnendseitig einer Fluidleitung 9 der Drosseleinheit 4 angeordnet. Die Fluidleitung 9 bildet eine fluidleitende Verbindung zwischen dem zweiten Kupplungselement 8 und dem Drosselelement 7 aus.

Das erste Kupplungselement 5 und das zweite Kupplungselement 8 sind vorliegend derart komplementär gestaltet, dass eine mittels der Kupplungselemente 5, 8 bewirkte fluidleitende Verbindung, wie sie anhand Fig. 1 ersichtlich ist, nicht zerstörungsfrei lösbar ist. Hierzu können die Kupplungselemente 5, 8 mit einer entsprechend gestalteten Rast- und/oder Schnappgeometrie versehen sein.

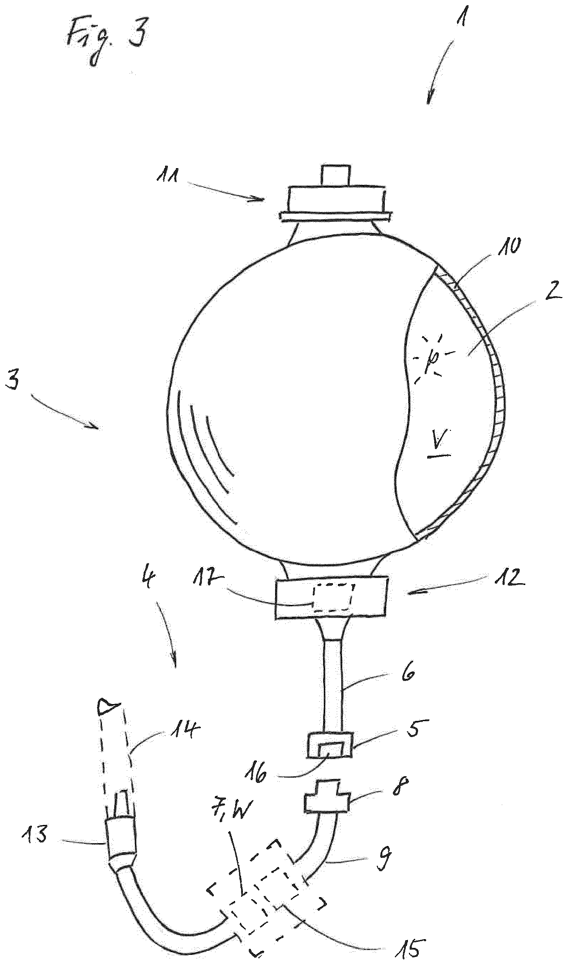

Weitere gegenständliche und funktionelle Merkmale der medizinischen Pumpvorrichtung 1 sind anhand der detaillierteren Darstellung der Fig. 3 ersichtlich. Dort ist erkennbar, dass die Pumpeinheit 3 eine elastomere Membran 10 aufweist, die das Pumpvolumen V ausbildet. Dabei weist die Pumpeinheit 3 einen Füllzustand auf, in dem das Pumpvolumen V mit dem medizinischen Fluid 2 befüllt ist. Das medizinische Fluid 2 ist vorliegend eine nicht näher bezeichnete Medikamentenflüssigkeit. In diesem Füllzustand ist die elastomere Membran 10 infolge einer mechanischen Einwirkung des medizinischen Fluids 2 ballonartig weichelastisch gedehnt. Dabei ist die elastomere Membran 10 anhand Fig. 3 aus zeichnerischen Gründen mit einer überhöhten Wandungsstärke dargestellt. Demgegenüber ist die elastomere Membran 10 in einem nicht mit medizinischen Fluid 2 befüllten Zustand schlaff oder jedenfalls weniger stark elastisch gedehnt. Zum Befüllen des Pumpvolumens V mit dem medizinischen Fluid 2 ist vorliegend ein wiederverschließbarer Einfüllstutzen 11 vorgesehen, der auf grundsätzlich bekannte Weise fluiddicht an die elastomere Membran 10 angeschlossen ist. Die elastisch gedehnte elastomere Membran 10 bewirkt einen Förderdruck p auf das Pumpvolumen V. Mittels des auf diese Weise bewirkten Förderdrucks p ist das medizinische Fluid 2 über einen auf grundsätzlich bekannte Weise fluiddicht an die elastomere Membran 10 angeschlossenen Durchlassstutzen 12 aus dem Pumpvolumen V in die Fluidleitung 6 förderbar. Die Fluidleitung 6 ist auf grundsätzlich bekannte Weise fluidleitend an den Durchlassstutzen 12 angeschlossen. Vorliegend ist die Fluidleitung in Form eines biegeflexiblen Schlauchabschnitts 6 ausgebildet, wobei das erste Kupplungselement 5 stirnendseitig an dem Schlauchabschnitt 6 angeordnet ist. Die Pumpeinheit 3 ist vorliegend nach Art einer medizinischen Elastomerpumpe ausgebildet. Solche Pumpen sind im Bereich der Medizintechnik als solche grundsätzlich bekannt und können auch als elastomere Infusionspumpen bezeichnet werden.

Zudem ist anhand Fig. 3 ersichtlich, dass die Fluidleitung 9 in Form eines biegeflexiblen Schlauchabschnitts ausgebildet ist. Dabei ist das zweite Kupplungselement 8 stirnendseitig des Schlauchabschnitts 9 angeordnet und in dem anhand Fig. 3 ersichtlichen Zustand nicht mit dem ersten Kupplungselement 5 verbunden. An einem dem zweiten Kupplungselement 8 abgewandten Stirnendbereich des Schlauchabschnitts 9 ist ein drittes Kupplungselement 13 angeordnet. Das dritte Kupplungselement 13 ist zur fluidleitenden Verbindung der Drosseleinheit 4 und - im fluidleitend miteinander verbundenen Zustand der Kupplungselemente 5, 8 - der medizinischen Pumpvorrichtung 1 mit einem zeichnerisch nicht näher dargestellten patientenseitigen Zugang eingerichtet. Vorliegend ist lediglich ein dem Patientenzugang zugeordneter Fluidleitungsabschnitt 14 strichliert und teilweise abgeschnitten angedeutet. Das dritte Kupplungselement 13 ist in Form eines Luer-Konnektors ausgebildet, wie er im Bereich der Medizintechnik grundsätzlich bekannt ist. Bei einer zeichnerisch nicht näher dargestellten Ausführungsform kann das dritte Kupplungselement 13 in Form eines NRFit-Konnektors ausgebildet sein. Weitere Komponenten der Drosseleinheit 4, insbesondere das Drosselelement 7, sind anhand Fig. 3 lediglich schematisch stark vereinfacht und insoweit strichliert dargestellt.

Wie weiter insbesondere anhand der Fig. 1 und 2 ersichtlich ist, weist die Drosseleinheit ein Filterelement 15 auf. Das Filterelement 15 ist der Fluidleitung 9 zugeordnet, wird bei einer Förderung des medizinischen Fluids 2 mit demselben durchspült und dient einer Filterung etwaiger partikelförmiger Verunreinigungen aus dem medizinischen Fluid 2.

Zudem weist die Pumpeinheit 3 - in dem anhand der Fig. 2 und 3 nicht mit der Drosseleinheit 4 verbunden Zustand - ein Versiegelungselement 16 auf. Das Versiegelungselement 16 ist vorliegend dem ersten Kupplungselement 5 zugeordnet, was jedoch nicht zwingend der Fall sein muss. Das Versiegelungselement 16 dient einer fluiddichten Versiegelung des Pumpvolumens V. Demnach ist das Pumpvolumen V in dem anhand der Fig. 2 und 3 ersichtlichen Zustand mittels des Versiegelungselements 16 öffenbar fluiddicht versiegelt. Das Versiegelungselement 16 ist vorliegend nach Art eines Stopfens ausgebildet, der stirnendseitig fluiddicht in das erste Kupplungselement 5 eingepasst ist. Bei einer zeichnerisch nicht näher dargestellten Ausführungsform kann das Versiegelungselement 16 auch derart gestaltet sein, dass das erste Kupplungselement 5 öffenbar stoffschlüssig fluiddicht mit dem Versiegelungselement 16 versiegelt ist. Vor einer manuellen Verbindung der Kupplungselemente 5, 8 wird das Versiegelungselement 16 manuell entfernt und somit das Pumpvolumen V entsiegelt.

Zudem weist die Pumpeinheit 3 einen Druckminderer 17 auf. Der Druckminderer 17 ist der Fluidleitung 6 zugeordnet und wird bei einer Förderung des medizinischen Fluids 2 mit demselben durchspült. Druckminderer sind im Bereich der Fluidtechnik als solche grundsätzlich bekannt. Dabei ist der Druckminderer 17 anhand der Figuren lediglich stark vereinfacht schematisch dargestellt. Anhand Fig. 3 ist gezeigt, dass der Druckminderer 17 vorliegend in den Durchlassstutzen 12 integriert ist. Dies ist jedoch nicht zwingend der Fall. Mittels des Druckminderers ist der Förderdruck p auf einen vorbestimmten, insbesondere konstanten, Druck reduzierbar.

Die medizinische Pumpvorrichtung 1 ist unter Verwendung des anhand der Prinzipskizze der Fig. 4 gezeigten Kits A modular ausgebildet.

Das Kit A weist eine Gruppe G1 mit mehreren unterschiedlichen Pumpeinheiten 3, 3', 3" auf. Die Pumpeinheiten 3, 3', 3" weisen unterschiedlich dimensionierte Pumpvolumina V, V', V" auf. Vorliegend besteht die Gruppe G1 aus drei unterschiedlichen Pumpeinheiten 3, 3', 3". Hiervon abweichend kann die Gruppe G1 auch aus lediglich zwei unterschiedlichen Pumpeinheiten oder wesentlich mehr als den gezeigten drei Pumpeinheiten 3, 3', 3" bestehen. Das Pumpvolumen V der Pumpeinheit 3 beträgt vorliegend 60 ml. Das Pumpvolumen V' der Pumpeinheit 3' beträgt vorliegend 120 ml. Das Pumpvolumen V" der Pumpeinheit 3" beträgt vorliegend 270 ml. Mit anderen Worten ist die Pumpeinheit 3 zur Verabreichung von 60 ml, die Pumpeinheit 3' von 120 ml und die Pumpeinheit 3" zur Verabreichung von 270 ml des medizinischen Fluids 2 eingerichtet. Im Übrigen gilt im Hinblick auf die gegenständliche und funktionelle Ausgestaltung der Pumpeinheiten 3' und 3" das zur Pumpeinheit 3 insbesondere in Zusammenhang mit den Fig. 1 bis 3 Offenbarte in entsprechender Weise, so dass zur Vermeidung von Wiederholungen auf die diesbezügliche Offenbarung verwiesen wird.

Das Kit A weist zudem eine Gruppe G2 mit mehreren unterschiedlichen Drosseleinheiten 4, 4', 4" auf. Die Drosseleinheiten 4, 4', 4" weisen unterschiedlich dimensionierte Drosselwirkungen W, W', W" auf. Vorliegend beträgt eine mit der Drosselwirkung W korrespondierende Flussrate 5 ml/h, eine mit der Drosselwirkung W' korrespondierende Flussrate 4 ml/h und eine mit der Drosselwirkung W" korrespondierende Flussrate 2 ml/h. Im Übrigen gilt im Hinblick auf die gegenständliche und funktionelle Gestaltung der Drosseleinheiten 4' und 4" das zur Drosseleinheit 4 Offenbarte in entsprechender Weise, so dass zur Vermeidung von Wiederholungen auf die diesbezügliche Offenbarung verwiesen wird. Die Gruppe G2 umfasst vorliegend die drei unterschiedlichen Drosseleinheiten 4, 4', 4". Hiervon abweichend kann die Gruppe G2 auch lediglich zwei unterschiedliche Drosseleinheiten oder deutlich mehr als die gezeigten drei Drosseleinheiten 4, 4', 4" aufweisen.

Bei einer nicht gezeigten Ausführungsform können die Drosselwirkungen der Drosseleinheiten unterschiedlich im Hinblick auf eine Toleranz und/oder Genauigkeit der erzielbaren Flussraten sein.

Die Pumpeinheiten 3, 3', 3" und die Drosseleinheiten 4, 4', 4" sind innerhalb des Kits A räumlich voneinander getrennt angeordnet, was anhand der strichlierten Linie L schematisch angedeutet ist. Demnach sind die Pumpeinheiten 3, 3', 3" und die Drosseleinheiten 4, 4', 4" innerhalb des Kits A nicht fluidleitend miteinander verbunden. Das Kit A weist vorliegend eine Verpackungsanordnung V auf, in der die Gruppen G1, G2 gemeinsam verpackt sind. Dies schließt nicht aus, dass die Gruppen G1, G2 und/oder die einzelnen Pumpeinheiten 3, 3', 3" und die einzelnen Drosseleinheiten 4, 4', 4" jeweils zusätzlich gesondert verpackt sind. Die Verpackungsanordnung V ist anhand Fig. 4 lediglich strichliert angedeutet.

Das Kit A dient einer endnutzerseitigen modularen Ausbildung insbesondere der medizinischen Pumpvorrichtung 1 nach den Fig. 1 bis 3. Dabei ermöglicht die vorliegende Gestaltung des Kits A die Ausbildung 9 unterschiedlicher Spezifikationen im Hinblick auf eine Kombination der Pumpvolumina V, V', V" und der Drosselwirkungen W, W', W" bzw. der jeweils hiermit korrespondierenden Flussraten. Dementsprechend ermöglicht das Kit A einem Endnutzer, beispielsweise einem Patienten oder medizinischen Personal, eine anwendungsfallgerechte modulare Ausbildung einer auf den spezifischen Anwendungsfall angepassten medizinischen Pumpvorrichtung. Denn je nachdem, ob der Anwendungsfall die Verabreichung einer geringeren oder größeren Menge des medizinischen Fluids 2 und eine kürzere oder längere Verabreichungsdauer erfordert, kann der Endnutzer dementsprechend hieran angepasst eine der Pumpeinheiten 3, 3', 3" mit einer der Drosseleinheiten 4, 4', 4" kombinieren und mittels der Kupplungselemente 5, 8 (vgl. Fig. 1 bis 3) miteinander verbinden. Beispielsweise ermöglicht die vorliegende Kombination des auf 60 ml bemessenen Pumpvolumens V und der auf eine Flussrate von 5 ml/h bemessenen Drosselwirkung W eine Infusionsdauer von 12 h. Ist hiervon abweichend beispielsweise eine Infusionsdauer von 15 h oder 30 h und hierbei eine Verabreichung von 60 ml des medizinischen Fluids 2 erforderlich, kann der Endnutzer die Pumpeinheit 3 mit der Drosseleinheit 4' bzw. der Drosseleinheit 4" kombinieren.

Bei der nicht gezeigten Ausführungsform kann eine anwendungsfallgerechte Auswahl im Hinblick auf die Genauigkeit der Drosselwirkung erfolgen. Erlaubt ein Anwendungsfall beispielsweise eine vergleichsweise große Schwankung der resultierenden Infusionsdauer, kann der Endnutzer eine Drosseleinheit mit vergleichsweise geringer Genauigkeit der erzielbaren Flussrate auswählen.

Anhand Fig. 5 ist ein Kit B gezeigt. Mittels des Kits B ist die medizinische Pumpvorrichtung 1 nach den Fig. 1 bis 3 modular ausbildbar. Hierzu weist das Kit B die Pumpeinheit 3 und die Drosseleinheit 4 auf. Zur Vermeidung von Wiederholungen wird auf das zu Kit A Offenbarte verwiesen, das in entsprechender Weise für Kit B gilt. Das Kit B unterscheidet sich lediglich dahingehend von dem Kit A, dass anstelle der Gruppen G1, G2 mit mehreren Pumpeinheiten bzw. Drosseleinheiten lediglich wenigstens die eine Pumpeinheit 3 und die wenigstens eine Drosseleinheit 4 umfasst ist.

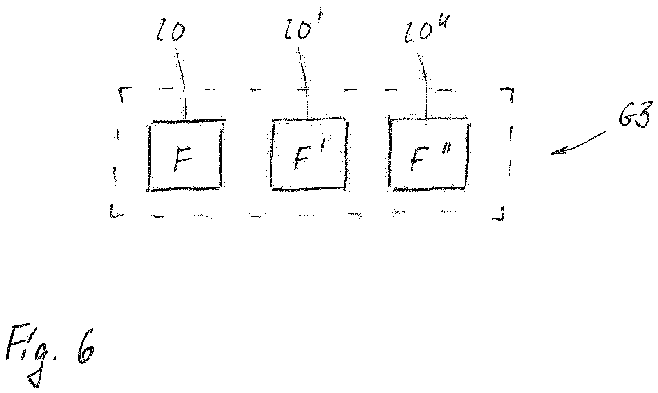

Anhand Fig. 6 ist eine Gruppe G3 mit mehreren unterschiedlichen Funktionseinheiten 20, 20', 20" ersichtlich. Die unterschiedlichen Funktionseinheiten 20, 20', 20" weisen unterschiedlich dimensionierte strömungsmechanische Eigenschaften F, F', F" und sind jeweils fluidleitend mit einer der Pumpeinheiten 3, 3', 3" und/oder einer der Drosseleinheiten 4, 4', 4" verbindbar. Demnach ist das anhand Fig. 4 ersichtliche Kit A vereinfacht ausgedrückt um die weitere Gruppe G3 erweiterbar.

## Patentansprüche

1. Kit (A) zur modularen Ausbildung einer medizinischen Pumpvorrichtung (1) zur Förderung eines medizinischen Fluids (2), aufweisend
- eine Gruppe (G1) mit mehreren unterschiedlichen Pumpeinheiten (3, 3', 3"), die jeweils ein Pumpvolumen (V, V', V") zur Aufnahme und Förderung des medizinischen Fluids (2) aufweisen,
- wobei die Pumpeinheiten (3, 3', 3") unterschiedlich dimensionierte Pumpvolumina (V, V', V") aufweisen,
- und wobei die Pumpeinheiten (3, 3', 3") jeweils ein erstes Kupplungselement (5) aufweisen, das derart gestaltet ist, dass das jeweilige Pumpvolumen (V, V', V") fluidleitend mit einer Drosseleinheit (4, 4', 4") verbindbar ist,
- und eine Gruppe (G2) mit mehreren unterschiedlichen Drosseleinheiten (4, 4', 4"), die jeweils zur Drosselung der Förderung des medizinischen Fluids (2) eingerichtet sind,
- wobei die Drosseleinheiten (4, 4', 4") unterschiedlich dimensionierte Drosselwirkungen (W, W', W") aufweisen,
- und wobei die Drosseleinheiten (4, 4', 4") jeweils ein zweites Kupplungselement (8) aufweisen, das derart gestaltet ist, dass die jeweilige Drosseleinheit (4, 4', 4") fluidleitend mit einem Pumpvolumen (V, V', V") einer der Pumpeinheiten (3, 3', 3") verbindbar ist,
- wobei die Pumpeinheiten (3, 3', 3") und die Drosseleinheiten (4, 4', 4") räumlich voneinander getrennt angeordnet sind.

2. Kit (A) nach Anspruch 1, **dadurch gekennzeichnet, dass** die Pumpeinheiten (3, 3', 3") jeweils eine elastomere Membran (10) aufweisen, die das Pumpvolumen (V, V', V") zur Aufnahme und Förderung des medizinischen Fluids (2) ausbildet und in einem wenigstens teilweise mit dem medizinischen Fluid (2) befüllten Zustand derart elastisch gedehnt ist, dass ein Förderdruck (p) auf das Pumpvolumen (V, V', V") bewirkt ist.

3. Kit (A) nach Anspruch 1 oder 2, **dadurch gekennzeichnet, dass** das erste Kupplungselement (5) und das zweite Kupplungselement (8) derart komplementär gestaltet sind, dass eine mittels der Kupplungselemente (5, 8) bewirkte fluidleitende Verbindung unlösbar ist.

4. Kit (A) nach einem der vorhergehenden Ansprüche, **dadurch gekennzeichnet, dass** die Drosseleinheiten (4, 4', 4") jeweils ein drittes Kupplungselement (13) aufweisen, das zur fluidleitenden Verbindung der Drosseleinheit (4, 4', 4") und damit der medizinischen Pumpvorrichtung (1) mit einem patientenseitigen Zugang eingerichtet ist.

5. Kit (A) nach einem der vorhergehenden Ansprüche, **dadurch gekennzeichnet, dass** die Drosseleinheiten (4, 4', 4") jeweils ein Filterelement (15) aufweisen, das zur Filterung des medizinischen Fluids (2) vorgesehen ist.

6. Kit (A) nach einem der vorhergehenden Ansprüche, **dadurch gekennzeichnet, dass** die Pumpeinheiten (3, 3', 3") jeweils einen Füllzustand aufweisen, in dem das jeweilige Pumpvolumen (V, V', V") mit dem medizinischen Fluid (2) vorbefüllt ist.

7. Kit (A) nach Anspruch 6, **dadurch gekennzeichnet, dass** die Pumpeinheiten (3, 3', 3") jeweils ein Versiegelungselement (16) aufweisen, mittels dessen das jeweilige Pumpvolumen (V, V', V") öffenbar fluiddicht versiegelt ist.

8. Kit (A) nach einem der vorhergehenden Ansprüche, **dadurch gekennzeichnet, dass** die Pumpeinheiten (3, 3', 3") jeweils einen Druckminderer (17) aufweisen, mittels dessen ein Förderdruck (p) des medizinischen Fluids (2) reduzierbar ist.

9. Kit (A) nach einem der vorhergehenden Ansprüche, **dadurch gekennzeichnet, dass** eine weitere Gruppe (G3) mit mehreren unterschiedlichen Funktionseinheiten (20, 20', 20") vorgesehen ist, wobei die Funktionseinheiten (20, 20', 20") unterschiedlich dimensionierte strömungsmechanische Eigenschaften (F, F', F") aufweisen, und wobei die Funktionseinheiten (20, 20', 20") jeweils fluidleitend mit einer der Pumpeinheiten (3, 3', 3") und/oder einer der Drosseleinheiten (4, 4', 4") verbindbar sind.

10. Medizinische Pumpvorrichtung (1) zur Förderung eines medizinischen Fluids (2), **gekennzeichnet durch** eine modulare Ausbildung unter Verwendung eines Kits (A) nach einem der Ansprüche 1 bis 9.

## Claims

1. Kit (A) for the modular configuration of a medical pump device (1) for delivering a medical fluid (2), having
- a group (G1) with a plurality of different pump units (3, 3', 3"), which each have a pump volume (V, V', V") for receiving and delivering the medical fluid (2),
- wherein the pump units (3, 3', 3") have differently dimensioned pump volumes (V, V', V"),
- and wherein the pump units (3, 3', 3") each have a first coupling element (5), which is designed in such a way that the respective pump volume (V, V', V") is connectable to a throttle unit (4, 4', 4") in a fluid-conducting manner,
- and a group (G2) with a plurality of different throttle units (4, 4', 4"), which are each designed to throttle the delivery of the medical fluid (2),
- wherein the throttle units (4, 4', 4") have differently dimensioned throttle effects (W, W', W"),
- and wherein the throttle units (4, 4', 4") each have a second coupling element (8), which is designed in such a way that the respective throttle unit (4, 4', 4") is connectable in a fluid-conducting manner to a pump volume (V, V', V") of one of the pump units (3, 3', 3"),
- wherein the pump units (3, 3', 3") and the throttle units (4, 4', 4") are arranged spatially separated from each other.

2. Kit (A) according to Claim 1, **characterized in that** the pump units (3, 3', 3") each have an elastomer membrane (10), which forms the pump volume (V, V', V") for receiving and delivering the medical fluid (2) and, in a state at least partially filled with the medical fluid (2), is elastically extended in such a way that a delivery pressure (p) on the pump volume (V, V', V") is produced.

3. Kit (A) according to Claim 1 or 2, **characterized in that** the first coupling element (5) and the second coupling element (8) are designed complementing each other in such a way that a fluid-conducting connection produced by means of the coupling elements (5, 8) is non-releasable.

4. Kit (A) according to one of the preceding claims, **characterized in that** the throttle units (4, 4', 4") each have a third coupling element (13), which is designed to connect the throttle unit (4, 4', 4"), and thus the medical pump device (1), in a fluid-conducting manner to a patient-side access.

5. Kit (A) according to one of the preceding claims, **characterized in that** the throttle units (4, 4', 4") each have a filter element (15), which is provided for filtering the medical fluid (2).

6. Kit (A) according to one of the preceding claims, **characterized in that** the pump units (3, 3', 3") each have a filling state in which the respective pump volume (V, V', V") is pre-filled with the medical fluid (2).

7. Kit (A) according to Claim 6, **characterized in that** the pump units (3, 3', 3") each have a sealing element (16), by means of which the respective pump volume (V, V', V") is openably sealed in a fluid-tight manner.

8. Kit (A) according to one of the preceding claims, **characterized in that** the pump units (3, 3', 3") each have a pressure reducer (17), by means of which a delivery pressure (p) of the medical fluid (2) is reducible.

9. Kit (A) according to one of the preceding claims, **characterized in that** a further group (G3) with a plurality of different functional units (20, 20', 20") is provided, wherein the functional units (20, 20', 20") have differently dimensioned properties (F, F', F") in terms of flow mechanics, and wherein the functional units (20, 20', 20") are each connectable in a fluid-conducting manner to one of the pump units (3, 3', 3") and/or one of the throttle units (4, 4', 4").

10. Medical pump device (1) for delivering a medical fluid (2), **characterized by** a modular configuration using a kit (A) according to one of Claims 1 to 9.

## Revendications

1. Kit (A) pour la formation modulaire d'un dispositif de pompage médical (1) servant au refoulement d'un fluide médical (2), présentant
- un groupe (G1) doté de plusieurs unités de pompage (3, 3', 3") différentes, qui présentent respectivement un volume de pompage (V, V', V") pour la réception et le refoulement du fluide médical (2),
- les unités de pompage (3, 3', 3") présentant des volumes de pompage (V, V', V") dimensionnés différemment,
- et les unités de pompage (3, 3', 3") présentant respectivement un premier élément d'accouplement (5) qui est configuré de telle sorte que le volume de pompage (V, V', V") respectif puisse être relié de manière fluidique à une unité d'étranglement (4, 4', 4"),
- et un groupe (G2) doté de plusieurs unités d'étranglement (4, 4', 4") différentes, qui sont conçues respectivement pour l'étranglement du refoulement du fluide médical (2),
- les unités d'étranglement (4, 4', 4") présentant des effets d'étranglement (W, W', W") dimensionnés différemment,
- et les unités d'étranglement (4, 4', 4") présentant respectivement un deuxième élément d'accouplement (8) qui est configuré de telle sorte que l'unité d'étranglement (4, 4', 4") respective puisse être reliée de manière fluidique à un volume de pompage (V, V', V") de l'une des unités de pompage (3, 3', 3"),
- les unités de pompage (3, 3', 3") et les unités d'étranglement (4, 4', 4") étant disposées de manière séparée spatialement les unes des autres.

2. Kit (A) selon la revendication 1, **caractérisé en ce que** les unités de pompage (3, 3', 3") présentent respectivement une membrane élastomère (10) qui forme le volume de pompage (V, V', V") pour la réception et le refoulement du fluide médical (2) et qui, dans un état rempli au moins partiellement avec le fluide médical (2), est étirée élastiquement de telle sorte qu'une pression de refoulement (p) sur le volume de pompage (V, V', V") soit produite.

3. Kit (A) selon la revendication 1 ou 2, **caractérisé en ce que** le premier élément d'accouplement (5) et le deuxième élément d'accouplement (8) sont configurés de manière complémentaire de telle sorte qu'une liaison fluidique produite au moyen des éléments d'accouplement (5, 8) ne soit pas libérable.

4. Kit (A) selon l'une des revendications précédentes, **caractérisé en ce que** les unités d'étranglement (4, 4', 4") présentent respectivement un troisième élément d'accouplement (13) qui est conçu pour la liaison fluidique de l'unité d'étranglement (4, 4', 4") et par conséquent du dispositif de pompage médical (1) à un accès côté patient.

5. Kit (A) selon l'une des revendications précédentes, **caractérisé en ce que** les unités d'étranglement (4, 4', 4") présentent respectivement un élément filtre (15) qui est prévu pour la filtration du fluide médical (2).

6. Kit (A) selon l'une des revendications précédentes, **caractérisé en ce que** les unités de pompage (3, 3', 3") présentent respectivement un état de remplissage dans lequel le volume de pompage (V, V', V") respectif est pré-rempli avec le fluide médical (2).

7. Kit (A) selon la revendication 6, **caractérisé en ce que** les unités de pompage (3, 3', 3") présentent respectivement un élément de scellement (16) au moyen duquel le volume de pompage (V, V', V") respectif est scellé de manière étanche aux fluides et ouvrable.

8. Kit (A) selon l'une des revendications précédentes, **caractérisé en ce que** les unités de pompage (3, 3', 3") présentent respectivement un réducteur de pression (17) au moyen duquel une pression de refoulement (p) du fluide médical (2) peut être réduite.

9. Kit (A) selon l'une des revendications précédentes, **caractérisé en ce qu'**un autre groupe (G3) doté de plusieurs unités fonctionnelles (20, 20', 20") différentes est prévu, les unités fonctionnelles (20, 20', 20") présentant des propriétés (F, F', F") de mécanique des fluides dimensionnées différemment, et les unités fonctionnelles (20, 20', 20") pouvant être reliées respectivement de manière fluidique à l'une des unités de pompage (3, 3', 3") et/ou à l'une des unités d'étranglement (4, 4', 4").

10. Dispositif de pompage médical (1) servant au refoulement d'un fluide médical (2), **caractérisé par** une formation modulaire à l'aide d'un kit (A) selon l'une des revendications 1 à 9.
